Europäisches Patentamt

European Patent Office

Office européen des brevets

(19)

(11) Veröffentlichungsnummer: **0 014 503**
B1

(12) **EUROPÄISCHE PATENTSCHRIFT**

(45) Veröffentlichungstag der Patentschrift:
28.07.82

(51) Int. Cl.³: **C 09 B 67/42,** C 09 B 67/18,
C 09 D 17/00, D 06 L 3/12

(21) Anmeldenummer: **80200073.7**

(22) Anmeldetag: **28.01.80**

(54) Verfahren zur Herstellung von in wässrigen Medien dispergierbaren, hochkonzentrierten, feindispersen, dispergiermittelarmen oder dispergiermittelfreien Präparaten von in Wasser schwer löslichen bis unlöslichen Wirksubstanzen in fester Form.

(30) Priorität: **02.02.79 CH 1057/79**

(43) Veröffentlichungstag der Anmeldung:
**20.08.80 Patentblatt 80/17**

(45) Bekanntmachung des Hinweises auf die Patenterteilung:
**28.07.82 Patentblatt 82/30**

(84) Benannte Vertragsstaaten:
**DE FR GB IT**

(56) Entgegenhaltungen:
**AT-A-189 308**
**CH-A-300 596**
**DE-A-2 817 453**
**FR-A-1 427 492**
**FR-A-2 049 897**
**FR-A-2 311 826**
**FR-A-2 323 435**
**FR-A-2 357 615**
**FR-A-2 388 029**
**GB-A-2 004 922**

(73) Patentinhaber: **Rohner AG Pratteln, Gempenstrasse 6,
CH-4133 Pratteln (CH)**

(72) Erfinder: **Wegmann, Jacques, Dr., Bückenweg 22,
CH-4126 Bettingen (CH)**

(74) Vertreter: **Becher, Pauline, Dr. et al, A. Braun, Braun,
Héritier, Eschmann AG Patentanwälte
Holbeinstrasse 36-38, CH-4051 Basel (CH)**

# Verfahren zur Herstellung von in wässrigen Medien dispergierbaren, hochkonzentrierten, feindispersen, dispergiermittelarmen oder dispergiermittelfreien Präparaten von in Wasser schwer löslichen bis unlöslichen Wirksubstanzen in fester Form

Die vorliegende Erfindung bezieht sich auf ein Verfahren zur Herstellung von in wässrigen Medien leicht und vollständig dispergierbaren, wenig oder kein Dispergiermittel enthaltenden, hochkonzentrierten, in fester Form als Pulver oder Granulate vorliegenden, feindispersen Präparaten von in Wasser schwer löslichen bis unlöslichen Wirksubstanzen, die zur Herstellung von Dispersionen der Wirksubstanzen in wässrigen oder wässrig-organischen Medien verwendet werden können. Als Wirksubstanzen kommen z.B. Insektizide, Herbizide, Pharmaka, Textilhilfsmittel, Textilveredlungsmittel und Kunststoffadditive, insbesondere aber natürliche oder künstliche Farbstoffe oder deren Vorprodukte, optische Aufheller und Pigmente in Frage.

Die erfindungsgemäss hergestellten Präparate können ohne Anwendung besonderer Massnahmen oder Zusätze durch einfaches Einrühren in wässrigen Medien dispergiert werden, wobei feindisperse und stabile, verdünnte oder konzentrierte Dispersionen der Wirkstoffe erhalten werden können.

Feste Präparate aus Farbstoffen oder Pigmenten und in Wasser unlöslichen Säureharzen sind bekannt. Beispielsweise sind in der DE-A-26 06 212 und in der DE-B-1 469 724 (die der GB-A-1 108 076 entspricht) derartige Präparate, die in organischen Medien leicht dispergierbar sind, beschrieben.

In der DE-A-28 17 453 wird ein Verfahren zur Herstellung von in wässrigen Medien dispergierbaren, als Pulver oder Granulate vorliegenden Präparaten beschrieben, die als Trägersubstanzen in Wasser schwer lösliche bis unlösliche, saure Gruppen enthaltende, mit Basen in eine wasserlösliche Form überführbare hochmolekulare organische Verbindungen enthalten und die schneller und leichter dispergierbar sind als die oben erwähnten Präparate. Auch diese Präparate haben den grundsätzlichen Nachteil, dass sie in einem Überschuss einer relativ konzentrierten Alkalilösung dispergiert werden müssen, was je nach dem Anwendungszweck der Dispersion als störend empfunden wird. Eine nachträgliche Neutralisation des vorhandenen überschüssigen Alkalis ist jedoch nicht einfach und kann die Qualität der Dispersion verschlechtern; daher wird diese Massnahme als unerwünschte Komplikation angesesen, die die Anwendungsmöglichkeiten der Präparate beschränkt.

Die erfindungsgemäss herstellbaren Präparate haben nicht die oben erwähnten Nachteile, weisen aber zusätzlich zu den Vorteilen der bekannten Präparationen noch weitere Vorteile auf.

Auch feste, reversibel dispergierbare Präparate von Wirksubstanzen, wie z.B. Farbstoffe und Pigmente, die frei von Dispergiermitteln sind und lediglich eine wasserlösliche, hochmolekulare Trägersubstanz enthalten, sind schon vorgeschlagen worden. Unter «reversibel dispergierbar» ist zu verstehen, dass die Dispersionen, die durch Einrühren eines festen Präparates in ein flüssiges Medium erhalten werden, den gleichen Feinheitsgrad haben wie die flüssige Dispersion, aus der das Präparat hergestellt wurde. In der GB-A-1 176 217 sind Präparate dieses Typs, die als Trägersubstanz bestimmte Cellulosederivate enthalten, beschrieben. Diese Trägersubstanzen können entweder in wässrigen oder in organischen Medien löslich sein. Von den Löslichkeitseigenschaften der Trägersubstanzen hängt es ab, ob die Präparate in wässrigen oder in organischen Medien dispergierbar sind. In wässrigen Medien dispergierbare Präparate, die wasserlösliche Trägersubstanzen auf Cellulosebasis enthalten, haben sich aber in der Technik nicht durchsetzen können. Dies beruht vermutlich darauf, dass redispergierbare Präparate relativ grosse Mengen Trägersubstanz enthalten müssen und dass die Trägersubstanz in wässrigem Medium nur langsam quillt, so dass sich die Präparate nur nach längerem (beispielsweise einstündigem) Rühren lösen.

Demgegenüber können die bekannten Präparate auf Basis wasserlöslicher, niedermolekularer Dispergiermittel, wie Ligninsulfonate oder Kondensationsprodukte von Napthalinsulfonsäuren mit Formaldehyd, durch blosses Einrühren in Wasser rasch und vollständig dispergiert werden. Sie haben aber bekanntlich eine Reihe von anderen Nachteilen. Insbesondere wird das Abwasser durch den hohen Gehalt derartiger Präparate an zum Teil stark schäumenden Dispergiermitteln belastet. Ausserdem beeinflussen diese Dispergiermittel verschiedene Anwendungen der Präparate in unerwünschtem Sinne. Bei der textilen Applikation von Farbstoffen, sei es durch Färbung oder durch Druck, üben sie beispielsweise eine störende zurückhaltende Wirkung aus und begünstigen die unerwünschte Migration des Farbstoffes beim Trocknen der Textilien, d.h. sie halten den Farbstoff entweder im Färbebad oder bei der Foulardfärbung auf der Oberfläche der Textilien zurück, erschweren das Auswaschen von nicht fixierten Farbstoff oder verursachen die Anfärbung der weissen Stellen von Drucken, wenn sie in den üblichen Mengen verwendet werden. Sofern die Dispergiermittel im zu behandelnden Substrat verbleiben, was vor allem bei der Anwendung in der Masse (beispielsweise bei der Spinnfärbung) der Fall ist, können sie zudem die Echtheiten der Färbungen beeinflussen oder zur Vergilbung der Substrate führen. Bei der Anwendung von Pflanzenschutzmitteln rufen die Dispergiermittel die Bildung von wenig wasserbeständigen, durch Regen leicht abspülbaren, schlecht haltbaren und deshalb wenig wirksamen Überzügen auf den behandelten Pflanzenteilen hervor.

Die erfindungsgemäss herstellbaren Präparate haben nicht die genannten Nachteile, ohne dass deshalb andere Nachteile in Kauf genommen werden müssten. Wenn die Präparate für pharmazeutische Zwecke bestimmt sind, haben sie zudem den Vorteil, dass man Trägersubstanzen verwenden kann, von denen bekannt ist, dass sie physiologisch unbedenklich sind, und die schon zum Überziehen von Tabletten mit einer Schutzschicht oder für das Bedrucken von Lebensmittelpackungen verwendet werden.

Es ist auch schon vorgeschlagen worden, sowohl von Trägersubstanzen als auch von Dispergiermitteln freie, nur die Wirksubstanz enthaltende Präparate herzustellen, beispielsweise durch Nassgranulierung in einem Mehrphasensystem; dabei sollen Präparate erhalten werden, die sich sowohl in wässrigen als auch in organischen Medien leicht und vollständig dispergieren lassen. Präparate dieses Typs sind beispielsweise in der DE-A-24 59 457 (die der GB-A-1 491 736 entspricht) beschrieben. Soweit diese Präparate wasserunlösliche Wirksubstanzen enthalten, konnten die obigen Angaben allerdings nicht bestätigt werden.

Aus der FR-A-2 388 029 sind wässrige Färbepräparate bekannt, die mindestens einen in Wasser unlöslichen Farbstoff, ein Dispergiermittel, mindestens ein Additiv und ein in Wasser lösliches Polymerisationsprodukt, das Carboxylgruppen aufweist, enthalten. Diese Präparate zeigen nach dem Trocknen eine gute Redispergierbarkeit in Wasser. Die Polymerisationsprodukte können als wasserlösliche Salze vorliegen. Die flüssigen Präparate werden durch Mischen der Bestandteile unter Rühren, anschliessendes Homogenisieren und Mahlen erhalten.

Die FR-A-2 357 615 beschreibt feste Pigmentpräparate für Drucktinten, die 10 bis 90 Gew.-% eines Pigmentes und 90 bis 10 Gew.-% eines Polyacrylharzes mit Carboxylgruppen enthalten. Die Präparate können durch Zugabe des Pigmentes zu einer alkalischen Lösung des Polyacrylharzes und Ausfällen durch Ansäuern, durch Zerstäubungstrocknung einer Lösung von Pigment und Polyacrylharz oder durch Mischen des in Wasser unlöslichen Polyacrylharzes mit dem Pigment in einem Kneter hergestellt werden.

Aus der FR-A-1 427 492 ist ein Verfahren zur Herstellung einer Dispersion eines festen, teilchenförmigen Materials in einem wässrigen Medium bekannt, bei dem das Material mit einer wässrigen Lösung eines organischen Polymeren behandelt und das behandelte Material getrocknet und danach mit dem Medium gemischt wird. Durch diese Oberflächenbehandlung wird die Dispergierbarkeit in verschiedenen wässrigen Medien verbessert. Man verwendet niedrigmolekulare organische Polymere oder Copolymere in Mengen von 5 Gew.-% oder weniger, bezogen auf das zu behandelnde Material.

Die FR-A-2 049 897 bezieht sich auf Präparate, die mindestens einen in Wasser unlöslichen Farbstoff und ein hauptsächlich Styrol und Maleinsäureanhydrid oder seine in Wasser unlöslichen Derivate enthaltendes Copolymer, das unter der Einwirkung von Basen wasserlöslich wird, enthält. Die Präparate werden durch inniges Mischen der Bestandteile, beispielsweise durch gemeinsame trockene Mahlung oder Mahlung in Gegenwart einer Flüssigkeit in Mühlen oder Knetern, hergestellt.

Die CH-A-300 596 betrifft eine wässrige Pigmentpaste, die die Eigenschaften eines Dispergiermittels aufweist und neben Wasser ein pulvriges, wasserunlösliches Pigment und 1 bis 50 Gew.-%, bezogen auf das Pigment, eines Mischpolymeren aus Styrol, Maleinsäure-monomethylester und Maleinsäure-mono-sek.-butylester enthält. Die Pigmentpaste wird hergestellt, indem man das pulvrige, wasserunlösliche Copolymer zu einer wässrigen Aufschlämmung des Pigmentes zusetzt, das Mischpolymer gründlich mit dem Pigment vermengt und das Mischpolymer dann durch Zugabe einer bei Temperaturen unter 100°C flüchtigen Base löslich macht.

Es wurde nun gefunden, dass in wässrigen Medien dispergierbare, hochkonzentrierte, feindisperse, dispergiermittelarme oder dispergiermittelfreie Präparate von in Wasser schwer löslichen bis unlöslichen Wirksubstanzen in fester Form als Pulver oder Granulate, die

a) 40 bis 90 Gew.-%, vorzugsweise 50 bis 80 Gew.-%, mindestens einer in Wasser schwer löslichen bis unlöslichen Wirksubstanz mit einer mittleren Teilchengrösse unter 5 μm, vorzugsweise unter 2 μm,

b) 10 bis 60 Gew.-%, vorzugsweise 20 bis 50 Gew.-%, mindestens eines wasserlöslichen Salzes von Ammoniak oder einem flüchtigen Amin mit einem wasserunlöslichen Säureharz, vorzugsweise einem Maleinatharz, einem Maleinsäurecopolymeren oder einem Polymeren oder Copolymeren eines Maleinsäureabkömmlings, und

c) 0 bis 50 Gew.-% weitere Zusätze, wie Dispergiermittel, Netzmittel, Füllstoffe oder Coupagemittel oder Gemische davon,

enthalten, dadurch hergestellt werden können, dass man ein Gemisch aus Wirksubstanz(en) und Säureharz(en) in Gegenwart oder Abwesenheit von Wasser- oder Lösungsmittelresten den Dämpfen des Ammoniaks oder des flüchtigen Amins aussetzt, bis sich das wasserunlösliche Säureharz bzw. die wasserunlöslichen Säureharze vollständig in das wasserlösliche Salz bzw. die wasserlöslichen Salze umgewandelt hat bzw. haben, und dass man gegebenenfalls vorhandene Flüssigkeitsreste entfernt.

Die Herstellung von in neutralen wässrigen Medien redispergierbaren Präparaten durch Behandeln mit den Dämpfen von Ammoniak oder flüchtigen Aminen (nachfolgend als «Begasen» bezeichnet) ist im Stand der Technik nicht beschrieben. Sie ist aber nicht nur neu, sondern auch erfinderisch, denn es war nicht vorauszusehen, dass das Ammoniak bzw. die Amine beim Begasen leicht und vollständig mit den Säureharzen reagieren, so dass man vollständig redispergierbare Präparate erhält.

Die erfindungsgemäss hergestellten Präparate enthalten somit physiologisch und ökologisch unbedenkliche Trägersubstanzen und eignen sich für Applikationen in wässrigen Medien in feinst dispergierter Form. Sie können in Wasser schwer löslische bis unlösliche Wirksubstanzen aus einer ganzen Reihe von Verbindungsklassen enthalten, die auf diese Weise angewandt werden sollen; Beispiele für solche Wirksubstanzen sind Insektizide, Herbizide, Flammschutzmittel, Antioxidantien, Stabilisatoren, Kosmetika und Pharmaka, besonders aber natürliche Farbstoffe, wie Carotinoide, künstliche Farbstoffe oder Farbstoffbildner, wie deprotonierte basische Farbstoffe, Beizenfarbstoffe, Lösungsmittelfarbstoffe, Metallkomplexfarbstoffe, Dispersionsfarbstoffe, Naphtholfarbstoffe und Küpenfarbstoffe, anorganische Pigmente, wie Russ, Titandioxid, Eisenoxidhydrate, verschiedene Metallpulver, Chromoxid und Ultramarin, oder organische Pigmente, wie solche der Azo-, Anthrachinon-, Phthalocyanin-, Nitro-, Perinon-, Perylentetracarbonsäurediimid, Dioxazin-, Indolinon-, Imidazol-, Chinacridon-, Indigo- und Thioindigoreihe. Als Dispersionsfarbstoffe kommen speziell auch reaktive Dispersionsfarbstoffe und für den Thermotransferdruck, für Minimalauftragverfahren (Auftrag ohne Flottenüberschuss, der entfernt werden müsste) oder für das Auswaschen mit ganz wenig Wasser oder mit Lösungsmitteln bestimmte Dispersionsfarbstoffe in Frage, da die erfindungsgemäss hergestellten Präparate sich für diese neuen Verfahren ganz besonders gut eignen. Die Präparate können auch verschiedenartige Wirksubstanzen, z.B. wasserunlösliche Textilveredlungsmittel zusammen mit Farbstofffen oder Pigmenten oder Farbstoffe verschiedener Klassen, wie Küpenfarbstoffe zusammen mit Dispersionsfarbstoffen, oder Farbstoffe zusammen mit Pigmenten, enthalten.

Als wasserunlösliche Säureharze, deren wasserlösliche Salze mit Ammoniak oder einem flüchtigen Amin erfindungsgemäss als Trägersubstanzen verwendet werden, eignen sich ganz generell natürliche und künstliche Säureharze, wie sie in Karstens Lackrohstofftabellen, 4. Auflage (1967) bis 6. Auflage (1976), Verlag Curt R. Vincentz, Hannover, beschrieben sind. Besonders geeignet sind Reaktionsprodukte von Harzsäuren mit Maleinsäure und Polyalkoholen (wie Pentaerythrit), ferner freie saure Gruppen enthaltene Copolymere von Maleinsäure oder deren Derivaten (wie Maleinsäureanhydrid oder Maleinsäurehalbester) mit Olefinen (wie Äthylen, Propylen oder Butylen) oder anderen ungesättigten Verbindungen (wie Vinyläther, Vinylester, Vinylchlorid und Styrol). Insbesondere kommen teilweise veresterte Copolymere von Maleinsäure mit Styrol in Frage.

Die Säureharze sollen hochmolekular sein, d.h. Molekulargewichte über 500, vorzugsweise über 1000, haben. Molekulargewichte über 100 000 sind indessen in der Regel weniger geeignet, da bei Verwendung von wasserlöslichen Salzen von Säureharzen mit so hohen Molekulargewichten Präparate erhalten würden, die sich weniger leicht und schnell in wässrigen Medien dispergieren liessen und deren Dispersionen eine zu hohe Viskosität hätten. Die besten Resultate werden mit Mischungen von Säureharzen mit verschiedenen Molekulargewichten erzielt, denn Säureharze mit Molekulargewichten von 1000 bis 2000 haben die beste Wirkung als Mahlzusätze, während Säureharze mit Molekulargewichten von 20 000 bis 50 000 die besten Ergebnisse hinsichtlich der vollständig reversiblen Dispergierbarkeit liefern.

Als Amine können leicht flüchtige Amine, wie Äthylamin, Diäthylamin, Trimethylamin und Triäthylamin, verwendet werden.

Die erfindungsgemäss herstellbaren Präparate enthalten mindestens 10 Gew.-% der Trägersubstanz (Komponente b), damit sie einwandfrei reversibel dispergierbar sind. Mengen von mehr als 60 Gew.-% Trägersubstanz bringen keinen Vorteil. Wenn Präparate mit geringem Gehalt an Wirksubstanzen (Komponente a) erforderlich sind, können statt hoher Mengen Trägersubstanz auch zusätzlich zur Trägersubstanz bis zu 50 Gew.-% inerte, wasserlösliche Stellmittel (Komponente c) verwendet werden.

Ganz allgemein können die Präparate bis zu 50 Gew.-% weitere Zusätze enthalten. Beispiele solcher Zusätze sind die oben erwähnten wasserlöslichen Stellmittel, ferner andere Coupagemittel, Bindemittel, Netzmittel und Dispergiermittel. Wasserunlösliche Zusätze, wie Kreidemehl, Aluminiumoxidhydrat oder Teflonpulver, sollen mindestens den gleichen Feinheitsgrad (höchstens die gleiche mittlere Teilchengrösse) wie die Wirksubstanz haben. Beispiele von wasserlöslichen Coupagemitteln (Stellmitteln) sind wasserlösliche Amide, Hydroxyl- und Ätherverbindungen, wie Sorbit oder Harnstoff, und natürliche oder künstliche Polymere, wie Alginate, Polyamide oder Polymethacrylate. Falls die Trägersubstanz(en) (Komponente b) keine Netzwirkung haben, kann es von Vorteil sein, Netzmittel zuzusetzen, damit pulverförmige Präparate in Wasser rasch benetzt werden. Wenn die Ausgangsgemische durch Zerstäubungstrocknung hergestellt werden, können die Präparate gegebenenfalls bei der Mahlung der Wirksubstanz verwendete Dispergiermittel und/oder Netzmittel enthalten, beispielsweise Polyvinylpyrrolidon oder andere nichtionogene Dispergiermittel, wie Fettalkohol-Äthylenoxid-Kondensationsprodukte. Z.B. können die Präparate bis zu 10 Gew.-% anionische und/oder nichtionogene Dispergiermittel enthalten. Beim Mahlen der Wirksubstanz können aber auch wasserlösliche Salze von niederpolymeren Maleinatharzen oder Styrol-Maleinat-Copolymeren verwendet werden. In diesem Falle enthalten die Präparate überhaupt keine Dispergiermittel.

Das erfindungsgemässe Verfahren kann so ausgeführt werden, dass man zunächst nach an sich bekannten Methoden ein festes Präparat aus der Wirksubstanz und der erforderlichen Menge eines wasserunlöslichen Säureharzes herstellt und anschliessend das in dem festen Präparat enthaltene Säureharz durch Einwirkung von gasförmigem Ammoniak oder dem Dampf eines flüchtigen Amins in ein wasserlösliches Salz überführt. Überraschenderweise ist dies sogar bei getrockneten pulverförmigen oder granulierten Präparaten möglich. Die Salzbildung verläuft aber etwas schneller, wenn das Präparat, z.B. von der Herstellung her, noch Wasser oder Lösungsmittel enthält. Wenn man die Salzbildung durch Begasen auf einer Nutsche nach dem Abtrennen von einer Flüssigkeit durchführt, ist es allerdings erforderlich, dass das Präparat in lockerer, körniger Form und nicht etwa als kompakter Filterkuchen, der von den Dämpfen der Base nicht durchdrungen werden kann, vorliegt.

Die Herstellung der Präparate aus Wirksubstanz und wasserunlöslichem Säureharz, die als Ausgangsmaterial für die Begasung mit einer Base dienen, kann nach den bekannten Verfahren zur Herstellung solcher Präparationen erfolgen. Beispielsweise kann man das Lösungsmittel-Salzknetverfahren anwenden, das in der CH-A-533 669 ausführlich beschrieben ist. Man kann aber auch das weniger aufwendigere und rationellere Zweiphasenverfahren anwenden, das in der DE-A-28 17 453 beschrieben ist. Dieses Verfahren hat gegenüber der Anwendung von Knetern noch an den zusätzlichen Vorteil, dass das Säureharz keiner mechanischen Beanspruchung ausgesetzt und deshalb mit Sicherheit nicht geschädigt wird und dass die Zerkleinerung der Wirksubstanz auf die gewünschte mittlere Teilchengrösse in wässrigem oder in organischem Medium ausgeführt werden kann. Die Wahl des Zerkleinerungsmediums hängt nur davon ab, welche Variante vorteilhafter ist. Dies hängt von der Art der Wirksubstanz ab und kann durch Vorversuche leicht ermittelt werden.

Die nachträgliche Überführung des Säureharzes in ein wasserlösliches Salz ist deshalb universeller als das Verfahren, bei dem man bereits von einem wasserlöslichen Salz ausgeht, weil man die Wirksubstanz nicht nur in wässrigem, sondern auch in organischem Medium auf die gewünschte mittlere Teilchengrösse zerkleinern kann und weil man die Vereinigung der fein zerteilten Wirksubstanz mit dem wasserunlöslichen Säureharz und die anschliessende Überführung des Säureharzes in ein wasserlösliches Salz bei tiefen Temperaturen ohne Wärmezufuhr ausführen kann. Diese Faktoren sind vor allem von Bedeutung bei Wirksubstanzen, die nicht in wässrigem Medium gemahlen werden können, weil sie sich dabei entweder zersetzen oder in eine andere Modifikation übergehen würden, sowie bei Wirksubstanzen, die wärmeempfindlich sind und nicht einmal kurzfristig höheren Temperaturen ausgesetzt werden dürfen.

Die erfindungsgemäss hergestellten Präparate sind je nach dem Herstellungsverfahren feinpulverig bis körnig. Granulate haben in der Regel den Vorteil, dass sie freifliessend, rieselfähig und wenig stäubend sind.

Um die Präparate in Dispersionen überzuführen, genügt es in der Regel, sie mit Wasser zu übergiessen oder in Wasser einzurühren. Auf diese Weise werden bereits nach kurzer Zeit feindispergierte, homogene und von undispergierten bzw. ungelösten Rückständen freie Dispersionen erhalten. Je nach dem Anwendungszweck kann man diesen Dispersionen, die konzentriert oder verdünnt sein können, weitere Zusätze, wie Verdickungsmittel oder Dispergiermittel, zusetzen. Ein Zusatz von Dispergiermitteln ist beispielsweise erforderlich, wenn die Dispersionen besonders harten Bedingungen ausgesetzt werden sollen, wie sie beim Färben unter Druck bei hohen Temperaturen auftreten. Wenn die Dispersionen bei der Spinnfärbung von Viskosefasern angewandt werden sollen, ist es ebenfalls von Vorteil, zusätzlich anionische Dispergiermittel, wie Dinaphthylmethandisulfonate, zuzusetzen, um die Stabilität der Dispersion in Berührung mit der reduzierend wirkenden, natronalkalischen Cellulosexanthogenatlösung (Viskose) zu gewährleisten. In den meisten Fällen sind die erhaltenen Dispersionen jedoch ohne Anwendung zusätzlicher Mittel oder Massnahmen direkt verwendbar.

Die zahlreichen Anwendungen der aus den erfindungsgemäss hergestellten Präparaten erhältlichen Dispersionen, die an sich bekannt sind, wurden im Stand der Technik bereits so eingehend beschrieben, dass weitere Angaben nicht erforderlich sind.

Die Erfindung wird in den folgenden Beispielen näher erläutert. Teile bedeuten Gewichtsteile, und die Temperaturen sind in °C angegeben.

Beispiel 1

40 Teile eines Rohfarbstoffes, der durch Diazotieren eines Gemisches von o- und p-Äthylanilin und Kuppeln des so erhaltenen Gemisches von Diazoniumverbindungen mit 3-Hydroxy-2-naphthoesäure-2',5'-dimethoxyanilid erhalten wird, werden in Gegenwart von 1 Teil Polyvinylpyrrolidon und 1 Teil Dinaphthylmethandisulfonat in einer Rührwerkskugelmühle mit 300 Teilen Glaskugeln von 1 mm Durchmesser in 58 Teilen Wasser gemahlen, bis die mittlere Teilchengrösse des Farbstoffes weniger als 2 μm beträgt. Nach Abtrennen der Dispersion von den Glaskugeln wird sie durch Rühren oder Schütteln innig mit einer Lösung von 10 Teilen eines Styrol-Maleinat-Harzes mit einem durchschnittlichen Molekulargewicht von 40 000 in 80 Teilen wasserhaltigem Methyläthylketon vermischt und anschliessend mit Wasser nach und nach so weit verdünnt, dass sich das Präparat in Form eines Granulates abscheidet. Dann wird die lösungsmittelhaltige, wässrige Phase abgesaugt, das Präparat mit Wasser gewaschen und getrocknet. Das so erhaltene Granulat wird in einer Atmosphäre von Ammoniakdämpfen gelagert, bis keine Gewichtszunahme mehr festzustellen ist und eine Probe des Granulates sich in Wasser leicht und bei Raumtemperatur rasch und vollständig dispergieren lässt und beim Filtrieren der Dispersion durch ein Filterpapier kein Rückstand mehr festzustellen ist.

Ersetzt man die Ammoniakdämpfe durch die Dämpfe von Äthylamin, Diäthylamin oder Trimethylamin oder führt man die Begasung vor dem Trocknen des Granulates aus und trocknet erst anschliessend, so erhält man ähnlich gute Präparate.

Ersetzt man das genannten Styrol-Maleinat-Harz ganz oder teilweise durch ein Styrol-Maleinat-Harz mit einem mittleren Molekulargewicht von 2500, so resultieren ebenfalls gut dispergierbare Präparate, deren konzentrierte wässrige Dispersionen eine geringere Viskosität haben.

Die wässrigen Dispersionen eignen sich nach Zusatz von Dispiergiermitteln bzw. Verdickungsmitteln zum Färben bzw. Bedrucken von Synthesefasern nach üblichen Verfahren. Für Imprägnierverfahren können die wässrigen Dispersionen aber auch ohne Zusätze verwendet werden; dies erleichtert die Nachbehandlungsoperationen, wie Nachwäsche unter Verwendung von sehr wenig Wasser oder von Lösungsmitteln.

Beispiel 2

50 Teile 1-Anilino-3,5-di-(1-anthrachinonylamino)-triazin werden in Gegenwart von 1 Teil Dinaphthylmethandisulfonat in einer Rührwerkskugelmühle unter Verwendung von 300 Teilen Glaskugeln von 1 mm Durchmesser in 49 Teilen Wasser gemahlen, bis in der Dispersion keine Teilchen mit einer Grösse über 1 µm mehr vorhanden sind. Die von den Glaskugeln abgetrennte Dispersion wird filtriert und anschiessend mit einer Lösung von 12,5 Teilen eines Styrol-Maleinat-Harzes mit einem durchschnittlichen Molekulargewicht von 40 000 in 80 Teilen Methyläthylketon verrührt. Dann verdünnt man mit Wasser, bis ein gut filtrierbarer körniger Niederschlag entstanden ist, wäscht mit Wasser und trocknet. Das Granulat wird einer Ammoniakatmosphäre ausgesetzt, bis eine Probe in Wasser leicht und vollständig dispergiert werden kann. Erhöht man den Farbstoffgehalt im Präparat auf 90 Gew.-%, so erhält man ein ebenfalls noch gut dispergierbares Präparat, dessen Dispersionen eine geringere Viskosität haben.

Ersetzt man den oben genannten Küpenfarbstoff durch andere rohe Küpenfarbstoffe, wie z.B. Dichloringanthron, Pyranthron, Flavanthron, 1,5-Dibenzoylaminoanthrachinon oder gegebenenfalls halogeniertes Anthanthron oder Dibenzpyrenchinon, und verfährt im übrigen wie beschrieben, dann erhält man ähnlich gute Präparate.

Die Präparate sind leicht und vollständig verküpbar und lassen sich nach üblichen Verfahren auf Cellulosefasern applizieren. Für Mischgewebe können sie zusammen mit gemäss Beispiel 1 hergestellten Präparaten aus Dispersionsfarbstoffen appliziert werden. Beim Färben von Baumwolle-Polyester-Geweben nach dem Foulardverfahren ergeben sie ein egales Warenbild und zeigen eine geringere Migration als handelsübliche Pulverpräparate.

Verwendet man anstelle des Methyläthyletons eine gleiche Menge Pentoxon (4-Methoxy-4-methylpentanon-2), Methylacetoacetat, Äthylacetoacetat oder Propylencarbonat oder Gemische davon und verfährt im übrigen wie beschrieben, dann erhält man ebenfalls gute Präparate.

Ersetzt man ein Drittel des oben genannten Farbstoffs durch Colour Index Disperse Yellow 99, so erhält man ebenfalls ein gut verteilbares Präparat, das stabile, wässrige Dispersionen ergibt.

Beispiel 3

40 Teile 2-Hydroxyanthracen-3-carbonsäure-o-toluidid werden mit 10 Teilen eines Maleinatharzes, das durch Kondensieren von Abietinsäure mit Maleinsäure und Pentaerythrit gebildet wird, vermischt, mit Alkohol benetzt und in wässriger Natriumhydroxidlösung gelöst. Anschliessend wird die Lösung angesäuert und der sich bildende Niederschlag abfiltriert, gewaschen, getrocknet und in einer Atmosphäre von Ammoniakdämpfen behandelt, bis ein konstantes Gewicht erreicht ist. Das resultierende Präparat lässt sich in wässrigem Medium leicht dispergieren und unter Zusatz von Alkalihydroxiden mit Alkohol schnell und vollständig auflösen.

Stellt man aus den obigen Bestandteilen ein Präparat so her, wie es in Beispiel 1 der DE-A-28 17 453 beschrieben wurde, und behandelt es anschliessend mit Ammoniakdämpfen, dann resultiert ein rieselfähiges Granulat mit ähnlich guter Dispergierbarkeit.

Beispiel 4

40 Teile Pyranthron werden unter Zusatz von Natronlauge und Natriumdithionit in 1000 Teilen Wasser verküpt. Dann werden 10 Teile des Ammoniumsalzes eines Styrol-Maleinat-Harzes mit einem durchschnittlichen Molekulargewicht von 50 000 eingerührt. Nun leitet man Luft durch die Lösung und säuert anschliessend leicht an. Der entstandene Niederschlag wird abfiltriert und gewaschen. Vor oder nach dem Trocknen wird er mit 50 Teilen Natriumligninsulfonat vermischt, einer Atmosphäre von Ammoniakdämpfen ausgesetzt und gegebenenfalls noch vollständig getrocknet. Es resultiert ein gut dispergierbares, leicht zu verküpendes Pulver.

Ersetzt man das Ligninsulfonat durch eine gleiche Menge Harnstoff oder Sorbit oder einer Mischung dieser Coupagemittel, so erhält man ähnlich gute Präparate.

Verwendet man anstelle des Pyranthrons einen anderen Küpenfarbstoff und verfährt im übrigen wie beschrieben, so erhält man ebenfalls leicht zu verküpende Präparate.

## Patentansprüche

1. Verfahren zur Herstellung von in wässrigen Medien dispergierbaren, hochkonzentrierten, feindispersen, dispergiermittelarmen oder dispergiermittelfreien Präparaten von in Wasser schwer löslichen bis unlöslichen Wirksubstanzen in fester Form als Pulver oder Granulate, die

a) 40 bis 90 Gew.-% mindestens einer in Wasser schwer löslichen bis unlöslichen Wirksubstanz mit einer mittleren Teilchengrösse unter 5 μm,

b) 10 bis 60 Gew.-% mindestens eines wasserlöslichen Salzes von Ammoniak oder einem flüchtigen Amin mit einem wasserunlöslichen Säureharz und

c) 0 bis 50 Gew.-% weitere Zusätze enthalten, dadurch gekennzeichnet, dass man ein Gemisch aus Wirksubstanz(en) und Säureharz(en) in Gegenwart oder Abwesenheit von Wasser- oder Lösungsmittelresten den Dämpfen des Ammoniaks oder des flüchtigen Amins aussetzt, bis sich das wasserlösliche Säureharz bzw. die wasserunlöslichen Säureharze vollständig in das wasserlösliche Salz bzw. die wasserlöslichen Salze umgewandelt hat bzw. haben, und dass man gegebenenfalls vorhandene Flüssigkeitsreste entfernt.

2. Verfahren nach Anspruch 1, dadurch gekennzeichnet, dass man als Säureharz(e) Polymere oder Copolymere der Maleinsäure oder Polymere oder Copolymere von Abkömmlingen der Maleinsäure verwendet.

3. Verfahren nach Anspruch 1, dadurch gekennzeichnet, dass man als Säureharz(e) Harzsäuren und als flüchtige Base Ammoniak oder ein flüchtiges aliphatisches, alicyclisches oder aromatisches Amin verwendet.

4. Verfahren nach einem der Ansprüche 1 bis 3, dadurch gekennzeichnet, dass man als Wirksubstanz(en) wasserunlösliche Farbstoffe, Farbstoffvorprodukte, Pigmente oder optische Aufheller verwendet.

5. Verfahren nach einem der Ansprüche 1 bis 4, dadurch gekennzeichnet, dass man als weitere Zusätze Dispergiermittel und/oder Netzmittel und/oder Coupagemittel verwendet.

6. Verfahren nach Anspruch 5, dadurch gekennzeichnet, dass man als weitere Zusätze höchstens 10 Gew.-%, bezogen auf das Präparat, eines anionischen oder nichtionogenen Dispergiermittels oder einer Mischung davon verwendet.

7. Verfahren nach Anspruch 5 oder 6, dadurch gekennzeichnet, dass man als Coupagemittel wasserlösliche, in fester Form vorliegende Amide, Hydroxyl- oder Ätherverbindungen verwendet.

8. Verfahren nach einem der Ansprüche 1 bis 7, dadurch gekennzeichnet, dass man, bezogen auf das Präparat, 50 bis 80 Gew.-% Wirksubstanz(en) mit einer mittleren Teilchengrösse unter 2 μm und 20 bis 50 Gew.-% Säureharz(e), berechnet als Salz(e), verwendet.

## Claims

1. Process for preparing highly concentrated finely dispersed solid compositions of water insoluble to slightly soluble active compounds dispersible in an aqueous medium, containing little or no dispersing agent and having the form of powders or granulates and containing

a) 40 to 90% by weight of at least one active substance which is slightly soluble to insoluble in water and has an average particle size less than 5 μm,

b) 10 to 60% by weight of at least one water-soluble salt of ammonia or a volatile amine with an acid resin which is insoluble in water, and

c) 0 to 50% by weight of further additives, characterized in that a mixture of active substance(s) and acid resin(s) is exposed in the presence or absence of residues of water or solvents to the vapors of ammonia or the volatile amines until the water-insoluble acid resin or the water-insoluble acid resins is or are completely converted into the water-soluble salt or the water-soluble salts and in that any liquid residues which may be present are removed.

2. Process according to claim 1, characterized in that the acid resin(s) are polymers or copolymers of maleic acid or polymers or copolymers of derivatives of maleic acid.

3. Process according to claim 1 or 2, characterized in that the acid resin(s) are resin acids and the volatile base is ammonia or a volatile aliphatic, alicyclic or aromatic amine.

4. Process according to one of claims 1 through 3, characterized in that the active substance(s) are water-insoluble dyestuffs, dyestuff precursors, pigments or optical brighteners.

5. Process according to one of claims 1 through 4, characterized in that the further additives are dispersants and/or wetting agents and/or diluents.

6. Process according to claim 5, characterized in that the further additives are anionic or non-ionic dispersants or mixtures thereof in an amount of at most 10% by weight, based on the composition.

7. Process according to claim 5 or 6, characterized in that the diluents are water-soluble amides, hydroxy or ether compounds present in solid form.

8. Process according to one of claims 1 through 7, characterized by using, based on the composition, 50 to 80% by weight of active substance(s) having an average particle size less than 2 μm and 20 to 50% by weight of acid resin(s) calculated as salt(s).

## Revendications

1. Procédé de préparation de compositions solides dispersables en milieu aqueux de substances actives étant peu solubles à insolubles dans l'eau, fortement concentrées, finement dispersées, contenant peu ou pas d'agent dispersant, à l'état de poudres ou de granules qui contiennent

a) 40 à 90% en poids d'au moins une substance active peu soluble à insoluble dans l'eau d'une granulométrie moyenne inférieure à 5 μm,

b) 10 à 60% en poids d'au moins un sel soluble dans l'eau de l'ammoniac ou d'une amine volatile avec une résine acide insoluble dans l'eau, et

c) 0 à 50% en poids d'autres additifs, caractérisé en ce qu'on expose un mélange de la ou des substances actives et de la ou des résines acides, en présence ou en l'absence de résidus d'eau ou de solvants, aux vapeurs de l'ammoniac ou de l'amine volatile jusqu'à ce que la ou les résines acides insolubles dans l'eau soient complètement converties en le ou les sels solubles dans l'eau et on élimine les résidus de solvants éventuellement en présence.

2. Procédé suivant la revendication 1, caractérisé en ce qu'on utilise comme résine ou résines acides, des polymères ou copolymères de l'acide maléique ou des polymères ou copolymères de dérivés de l'acide maléique.

3. Procédé suivant la revendication 1, caractérisé en ce qu'on utilise comme résine ou résines acides, des acides résiniques et comme base volatile, de l'ammoniac ou une amine aliphatique, alicyclique ou aromatique volatile.

4. Procédé suivant l'une quelconque des revendications 1 à 3, caractérisé en ce qu'on utilise comme substance ou substances actives, des colorants, précurseurs de colorants, pigments ou azurants optiques insolubles dans l'eau.

5. Procédé suivant l'une quelconque des revendications 1 à 4, caractérisé en ce qu'on utilise comme autres additifs, des agents dispersants et/ou agents mouillants et/ou agents de coupage.

6. Procédé suivant la revendication 5, caractérisé en ce qu'on utilise comme autres additifs, au maximum 10% en poids, sur base de la composition, d'un agent dispersant anionique ou non ionique ou d'un mélange de tels agents.

7. Procédé suivant la revendication 5 ou 6, caractérisé en ce qu'on utilise comme agents de coupage, des amides, composés hydroxylés ou éthers solubles dans l'eau présentés sous forme solide.

8. Procédé suivant l'une quelconque des revendications 1 à 7, caractérisé en ce qu'on utilise, sur base de la composition, 50 à 80% en poids d'une ou plusieurs substances actives d'une granulométrie moyenne inférieure à 2 μm et 20 à 50% en poids d'une ou plusieurs résines acides, exprimé en sels.